# EUROPÄISCHE PATENTSCHRIFT

(11) **EP 1 396 278 B1**
(45) Veröffentlichungstag und Bekanntmachung des Hinweises auf die Patenterteilung: **14.06.2006**
(21) Anmeldenummer: 03019201.7
(22) Anmeldetag: 25.08.2003
(51) Int. Cl.: A61N 5/10

(54) **Isokinetische Gantry-Anordnung zur isozentrischen Führung eines Teilchenstrahls und Verfahren zu deren Auslegung**
Isokinetic gantry system for isocentric guiding of a particle beam and method therefor
Système de portique isocinétique pour le guidage isocentrique d'un faisceau de particules et procédé associé

(30) Priorität: 05.09.2002 DE 10241178
(43) Veröffentlichungstag der Anmeldung: 10.03.2004
(73) Patentinhaber: MT Aerospace AG, 86153 Augsburg (DE); Gesellschaft für Schwerionenforschung mit beschränkter Haftung, 64291 Darmstadt (DE)
(72) Erfinder: Kärcher, Hans, Dr., 61184 Karben (DE); Linn, Stefan, 64347 Griesheim (DE); Zimmerer, Thomas, 65474 Bischofsheim (DE); Koch, Dietmar, 55435 Gau-Algesheim (DE); Fuchs, Ralf Dr., 64846 Gross-Zimmemm (DE); Bourgeois, Walter, 69123 Heidelberg (DE); Spiller, Peter Dr., 61267 Neu-Anspach (DE)
(74) Vertreter: Grape, Knut

(56) Entgegenhaltungen:
- WO-A2-00/45891
- US-A- 4 628 523
- US-A- 4 917 344
- US-A- 6 158 708

## Beschreibung

Die Erfindung betrifft eine isokinetische Gantry-Anordnung zur isozentrischen Führung eines Teilchenstrahls und ein Verfahren zur Auslegung derselben.

Gantry-Anordnungen bzw. Gantry-Strukturen bzw. Gantries sind allgemein bekannt. Derartige Gantry-Anordnungen sind für die Medizin-Technik zur Behandlung von Menschen mit Protonen- bzw. Ionenstrahlen konzipiert. Dabei unterscheidet man Gantry-Anordnungen mit nicht-isozentrischer und isozentrischer Führung des Teilchenstrahls.

Bei der isozentrischen Führung lassen sich die Magnete (Bestrahlungsköpfe) um den Patienten kreisförmig, d.h. auf gleichem Radius, bewegen, so dass der Bestrahlungswinkel frei einstellbar ist. Die Ausrichtungen des Patienten auf dem in drei Raumrichtungen X-Y-Z bewegbaren und um die Hochachse Z drehbaren Patiententisch sind vergleichsweise klein, insbesondere in vertikaler Richtung.

Bei Gantry-Anordnungen mit nicht-isozentrischer Führung des Teilchenstrahls wird der zu behandelnde Patient gegenüber dem Teilchenstrahl im Allgemeinen wesentlich stärker ausgerichtet, d.h. unter psychischer Mehrbelastung in die jeweils gewünschte Stellung verbracht bzw. entsprechend den medizinischen Erfordernissen aus dieser in eine andere Stellung bewegt usw.

Bei den bekannten Protonen-Gantry-Anordnungen mit isozentrischer Führung des Teilchenstrahls kann der Teilchenstrahl auf einen ganz bestimmten (Behandlungs-)Zielpunkt gerichtet werden. Der Bestrahlungswinkel lässt sich mithin, wie in der konventionellen (Röntgen-, Gammaquanten-)Strahlentherapie, frei wählen und variieren. Der zu behandelnde Patient selbst kann während der gesamten Behandlungsdauer in ein und derselben Relativlage zum Patiententisch verbleiben. Die dabei erforderlichen Verfahrwege des Tisches sind relativ klein, insbesondere in der als kritisch geltenden vertikalen Z-Richtung betragen diese nur wenige Zentimeter, so dass der Zugang zum Patienten durch den Arzt verhältnismäßig gut ist. Diese Gantry-Anordnungen dienen insoweit zum Tragen und Führen von schweren Magneten auf Kreisbahnen mit relativ großen Radien, die beispielsweise zwischen 2 und 6 Metern liegen. Dabei besteht insbesondere das Problem, dass die üblicherweise steifigkeitsdimensionierten Gantry-Anordnungen - je nach Position und (Dreh-)Winkellage der Magnete - jeweils unterschiedlichen Verschiebungen bzw. Verformungen bzw. Deformationen ausgesetzt sind. Diese wiederum wirken sich auf eine genaue Führung des Teilchenstrahls und damit auf die Zielgenauigkeit nachteilig aus. Bei alledem kommt hinzu, dass derartige Gantry-Anordnungen, nicht zuletzt aufgrund der hohen Anforderungen an die Zielgenauigkeit bei der Teilchenstrahlführung, eine ausgesprochen hohe Steifigkeit aufweisen müssen und somit zumeist äußerst schwere Gebilde mit großen Abmessungen darstellen.

Das Problem des hohen Struktur-Eigengewichtes wirkt sich aber gerade bei steifigkeitsdimensionierten Gantry-Anordnungen, welche zur Strahlen-Behandlung mit (schweren) Ionen vorgesehen sind, gegenüber steifigkeitsdimensionierten Gantry-Anordnungen, welche der Therapie mit Protonen (leichten Ionen) dienen, noch weitaus stärker aus, da deren Magnete und die zwischen den Magneten angeordneten Strahlführungselemente zum Teil um den Faktor 10 und mehr schwerer sind. Solche außerordentlich großen Struktureigengewichte wiederum verursachen Verschiebungen bzw. Verformungen bzw. Deformationen innerhalb der Gantry-Anordnungen, welche sich erneut nachteilig auf die geforderte Zielgenauigkeit auswirken, weil - wie Strukturanalysen zeigen - der Verformungsanteil aus dem Eigengewicht der Schwer-Ionen-Gantry größer wird.

Beispielsweise ist aus der US-PS 4,917,344 eine Gantry-Anordnung zur isozentrischen Führung eines Teilchenstrahls bekannt, die um eine horizontale Längsachse drehbar ist und eine Strahlenoptik, mit Magneten, welche den von einem Teilchenbeschleuniger axial eingeschossenen Teilchenstrahl radial und senkrecht zur horizontalen Längsachse ablenkt, sowie eine weitgehend rotationssymmetrische Primärstruktur aufweist.

Der vorliegenden Erfindung liegt daher die Aufgabe zugrunde, eine isokinetische Gantry-Anordnung zur isozentrischen Führung eines Teilchenstrahls zur Verfügung zu stellen, mit welcher sich die obigen Nachteile verhindern lassen, welche mithin besonders einfach ausgebildet ist, in allen Drehwinkellagen der Gantry-Anordnung eine ausgesprochen hohe Zielgenauigkeit des Teilchenstrahls ermöglicht, ohne dass speziell dafür eine (zudem ständige) Feldstärken-Nachjustierung der Magnete oder der Strahlführungselemente erforderlich ist, und zudem ein verhältnismäßig geringes Gewicht aufweist, sowie ein Verfahren zur Auslegung einer solchen Gantry-Anordnung bereitzustellen.

Diese Aufgabe wird auf überraschend einfache Weise in vorrichtungstechnischer Hinsicht durch die Merkmale des Anspruchs 1 gelöst.

Durch die erfindungsgemäße Ausgestaltung der Gantry-Anordnung zur isozentrischen Führung eines Teilchenstrahls nach den Merkmalen des Anspruchs 1, lässt sich eine besonders einfache isokinetische Gantry-Anordnung erzielen. Diese ist um eine horizontale Längsachse drehbar und weist eine Strahlenoptik, mit Magneten, auf, die in ihrer Gesamtwirkung den von einem Teilchenbeschleuniger axial eingeschossenen Teilchenstrahl im Ergebnis radial und senkrecht zur horizontalen Längsachse umlenkt. Ferner besitzt diese eine weitgehend rotationssymmetrische Primärstruktur und eine von der Primärstruktur abgestützte, die Magnete halternde weniger steife Sekundärstruktur, wobei die Sekundärstruktur eine Steifigkeit aufweist, die derart bemessen ist, dass vertikale Verschiebungen der Magnete infolge der Belastung der Gantry-Anordnung mit ihrem Gewicht in sämtlichen Drehwinkellagen im Wesentlichen gleich groß bzw. isokinetisch sind. Die Magnete sind auf Kreisbahnen um eine Drehachse, welche gegenüber der horizontalen Längsachse der Gantry-Anordnung im unbelasteten Zustand verschoben ist, bewegbar. Zu diesem Zweck werden die Magnete erfindungsgemäß auf Kreisbahnen geführt, die in Ebenen verlaufen, welche zur horizontalen Längsachse bzw. Drehachse senkrecht bzw. leicht dazu geneigt sind. Eingestellt werden die Kreisbahnen in einer computerunterstützten Entwurfsphase unter Zuhilfenahme einer Struktur-Deformations-Analyse durch gezielte Feindimensionierung der Strukturbauteile, insbesondere über die Wahl ihrer Querschnitte. Auf diese Weise wird in allen Drehwinkellagen der Gantry-Anordnung eine äußerst hohe Zielgenauigkeit des Teilchenstrahls ermöglicht, so dass diesbezüglich praktisch kein zusätzlicher Aufwand zur Kompensation von Zielungenauigkeiten erforderlich ist. Dazu zählen beispielsweise Hebelsysteme zum Gewichtsausgleich oder eine, zudem ständige Feldstärken-Nachjustierung der Magnete oder der Strahlführungselemente. Da weniger steife Strukturbauteile integriert sind (Sekundärstruktur) und damit von vornherein größere Verschiebungen bzw. Verformungen bzw. Deformationen als sonst üblich zugelassen werden, besitzt die erfindungsgemäße isokinetische Gantry-Anordnung darüber hinaus den wesentlichen Vorteil, einerseits wegen verbesserter Werkstoffausnutzung wesentlich leichter zu sein und andererseits eine relativ kleine Bauhöhe, d.h. verminderte Abmessungen, aufzuweisen. Beides wiederum fördert ebenfalls die Zielgenauigkeit des Teilchenstrahls. Zugleich tragen die weniger steif ausgeführten Strukturbereiche zu erheblichen Werkstoffeinsparungen bei. Mit der erfindungsgemäßen Gantry-Anordnung werden folglich Voraussetzungen für eine sehr gute isozentrische Führung des Teilchenstrahls verwirklicht, ohne dass die Struktur zu massiv und kostenaufwendig ausfällt. Die Gantry-Struktur nach der Erfindung eignet sich schließlich gleichermaßen für die Strahlführung von Protonen (leichten Ionen) als auch von (schweren) Ionen.

Vorteilhafte konstruktive Einzelheiten der erfindungsgemäßen Gantry-Anordnung sind in den Ansprüchen 2 bis 21 beschrieben.

In besonders vorteilhafter Weise ist entsprechend den Merkmalen nach Anspruch 2 vorgesehen, dass die Sekundärstruktur eine Steifigkeit aufweist, die derart bemessen ist, dass vertikale Verschiebungen der Magnete infolge ihres Gewichtes in sämtlichen Drehwinkellagen der Gantry-Anordnung gleich groß sind.

Eine vorteilhafte Ausgestaltung der erfindungsgemäßen Gantry-Anordnung besteht in der gemeinsamen Festlegung des Bestrahlungs-Zielpunktes und der durch Lasten verschobenen Drehachse, die je nach Winkellage der Gantry etwas im Raum wandert. Nach Anspruch 3 besteht diese darin, dass als Bestrahlungs-Zielpunkt der "Schnittpunkt" zwischen dem Ionen-/Protonenstrahl und der lastverschobenen theoretischen Drehachse dient, die gegenüber der horizontalen Längsachse der Gantry-Anordnung im unbelasteten Zustand mittels der Methode der kleinsten Fehlerquadrate aus Lastverschiebungen bestimmbar ist.

Weiterhin liegt es im Rahmen der Erfindung, die Primärstruktur entsprechend den Ansprüchen 4 bis 7 als ein Raumfachwerk aus im Wesentlichen horizontalen und vertikalen Trägern sowie diagonalen, zum Teil sich flächenzentrisch schneidenden, Trägern auszubilden und an deren Enden zwei, insbesondere kastenförmige, Auflagertragringe, die mit stationären Lagerständern zusammenwirken, vorzusehen. Dabei ist es zweckmäßig, einen der zwei stationären Lagerständer als Loslager und den anderen der zwei stationären Lagerständer als Festlager auszugestalten.

Von besonderer konstruktiver Bedeutung für eine Reduzierung von Gewichts- und Massenkräften einerseits und eine Erhöhung der Zielgenauigkeit des Teilchenstrahls andererseits sind die Maßnahmen des Anspruchs 8, wonach die von der Primärstruktur abgestützte, die Magnete halternde Sekundärstruktur gegenüber der Primärstruktur weniger steif ausgebildet ist.

In ganz vorteilhafter Ausgestaltung der erfindungsgemäßen Gantry-Anordnung ist die von der Primärstruktur abgestützte, die Magnete halternde Sekundärstruktur nach Anspruch 9 gegen azimutale Kippbewegungen der Magnete zusätzlich torsionssteif an der Primärstruktur angebunden, wodurch sich die Zielgenauigkeit des Teilchenstrahls noch weiter verbessern lässt.

Zusätzlich liegt es im Rahmen der Erfindung, die Gantry-Anordnung vorteilhafterweise mit einer von der Primärstruktur abgestützten Sekundärstruktur auszustatten, die nach den Ansprüchen 10 bis 17 wenigstens einen, insbesondere drei, Magnete aufnimmt, wobei sich zwischen den Magneten mehrere Strahlführungselemente, wie Steerer und Quadrupole, befinden, auf die jedoch nicht näher eingegangen wird, da diese - im Gegensatz zu den Magneten - das Verformungsverhalten der Gantry-Struktur weniger mitbeeinflussen.

In diesem Zusammenhang hat es sich als zweckmäßig erwiesen, den ersten Magneten nach den Maßnahmen des Anspruchs 13 auf der horizontalen Längsachse über lange Träger an die Primärstruktur freitragend bzw. fliegend anzuordnen und zum zweiten und dritten Magneten eine Anbindung über Stelzen herzustellen, die dazu führt, dass die "großen" Bewegungen des mittleren Gantry-Bereiches von den Magneten weitgehend isokinetisch mitgemacht werden.

Eine vorteilhafte Ausgestaltung der erfindungsgemäßen Gantry-Anordnung ergibt sich nach Anspruch 18, unter Verwendung von Magneten, die zur Aufnahme und zur Umlenkung eines Teilchenstrahls aus Protonen ausgebildet sind. Dementsprechend lässt sich die erfindungsgemäße Gantry-Anordnung mit Vorteil als sogenannte Protonen-Gantry einsetzen.

Alternativ dazu ist erfindungsgemäß von besonders großer Bedeutung, dass die Magnete entsprechend den Merkmalen des Anspruchs 19 zur Aufnahme und zur Umlenkung eines Teilchenstrahls aus Ionen ausgebildet sind. Insoweit lässt sich die erfindungsgemäße Gantry-Anordnung ganz bevorzugt als sogenannte Ionen-Gantry verwenden. Da die für die Strahlführung einer Ionen-Gantry erforderlichen Magnete und Teilchenstromführungselemente jedoch erheblich größer und schwerer als bei einer Protonen-Gantry sind, wirken sich die durch die Erfindung erzielbaren Verbesserungen wesentlich auf die Größen- und Abmessungsverhältnisse der Gantry-Anordnung insgesamt und den Materialaufwand aus und führen damit zu erheblichen Kosteneinsparungen.

Nach Anspruch 20 sind zwischen den Magneten Teilchenstrahlführungselemente angeordnet.

Zweckmäßigerweise bilden der zweite und dritte Magnet entsprechend den Maßnahmen des Anspruchs 21 eine integrierte Baueinheit.

Diese Aufgabe wird weiterhin in verfahrenstechnischer Hinsicht auf überraschend einfache Weise durch die Merkmale des Anspruchs 22 gelöst.

Demnach wird mittels des erfindungsgemäßen Verfahrens zur Auslegung einer Gantry-Anordnung mit isozentrischer Führung eines Teilchenstrahls die Steifigkeit einer im Wesentlichen rotationssymmetrischen Primärstruktur derart bemessen, dass die vertikalen Verschiebungen der Magnete infolge ihres Gewichtes in sämtlichen Drehwinkellagen der Gantry-Anordnung im Wesentlichen gleich groß, vorzugsweise gleich groß sind, wobei die Magnete auf Kreisbahnen um eine Drehachse, welche gegenüber der horizontalen Längsachse der Gantry-Anordnung im unbelasteten Zustand verschoben ist, bewegt werden. Auf diese Weise lässt sich eine Gantry-Anordnung erhalten, die einfach ausgestaltet ist, zugleich aber in allen Drehwinkellagen der Gantry-Anordnung eine äußerst hohe Zielgenauigkeit des Teilchenstrahls ermöglicht. Ein wesentlicher Aspekt bei dem erfindungsgemäßen Verfahren besteht darin, dass bei der Auslegung und Bemessung weitaus größere und damit von vornherein besser beeinflussbare Verschiebungen bzw. Verformungen bzw. Deformationen als sonst üblich zugelassen werden, die zu einer Gantry-Anordnung führen, welche aufgrund der verbesserten Werkstoffausnutzung wesentlich leichter und kleinbauender sowie damit einhergehend besonders kostengünstig herstellbar ist.

Schließlich liegt es in diesem Zusammenhang noch im Rahmen der Erfindung, die durch Lasten abgesenkte theoretische Drehachse nach Anspruch 23 gegenüber der horizontalen Längsachse der Gantry-Anordnung im unbelasteten Zustand mittels der Methode der kleinsten Fehlerquadrate zu bestimmen und als Bestrahlungs-Zielpunkt den "Schnittpunkt" zwischen dem Ionen-/Protonenstrahl und der lastverschobenen theoretischen Drehachse zu definieren.

Weitere Merkmale, Vorteile und Einzelheiten der Erfindung ergeben sich aus der nachfolgenden Beschreibung bevorzugter Ausführungsformen der Erfindung sowie anhand der Figuren. Hierbei zeigen:
- Fig. 1: eine schematische Seitenansicht einer Ausführungsform einer erfindungsgemäß ausgebildeten Gantry-Anordnung,
- Fig. 2: eine schematische Draufsicht auf die Ausführungsform der erfindungsgemäß ausgebildeten Gantry-Anordnung nach der Fig. 1,
- Fig. 3: eine Seitenansicht einer anderen Ausführungsform einer erfindungsgemäß ausgebildeten Gantry-Anordnung, in vergrößerter Darstellung,
- Fig. 4: eine Vorderansicht der Ausführungsform der erfindungsgemäß ausgebildeten Gantry-Anordnung nach der Fig. 3,
- Fig. 5A und 5B: schematische Querschnittansichten der Ausführungsform der erfindungsgemäß ausgebildeten Gantry-Anordnung nach den Fig. 3 und 4 in zwei unterschiedlichen Drehwinkellagen bzw. Stellungen der Gantry-Anordnung, und
- Fig. 6: ein schematisches Balkendiagramm zu der Ausführungsform der erfindungsgemäß ausgebildeten GantryAnordnung nach den Fig. 3 und 4 zur Darstellung von Verschiebungen bzw. Verformungen bzw. Deformationen, die durch das Gewicht der Magnete und/oder Teilchenstrahlführungselemente hervorgerufen werden.

In der nachfolgenden Beschreibung von Ausführungsformen einer erfindungsgemäßen Gantry-Anordnung 10 zur isozentrischen Führung eines Teilchenstrahls 12 sind einander entsprechende, gleiche Bauteile jeweils mit identischen Bezugsziffern versehen.

Bei den dargestellten Ausführungsformen handelt es sich um eine Gantry-Anordnung, die zur Strahlführung von Protonen, insbesondere schweren Ionen, vorgesehen ist. So erweist sich die Bestrahlung von Patienten mit Ionen, zum Beispiel vom Kohlenstoff, als eine erfolgversprechende Behandlung, die ein zielgenaues Ansteuern von tiefliegenden Tumoren und deren Abtötung erlaubt, wobei das angrenzende, außerhalb des Tumorvolumens liegende gesunde Gewebe, weitestgehend verschont bleibt. Schwer-Ionenstrahlen sind dabei gegenüber Protonenstrahlen biologisch wesentlich effizienter.

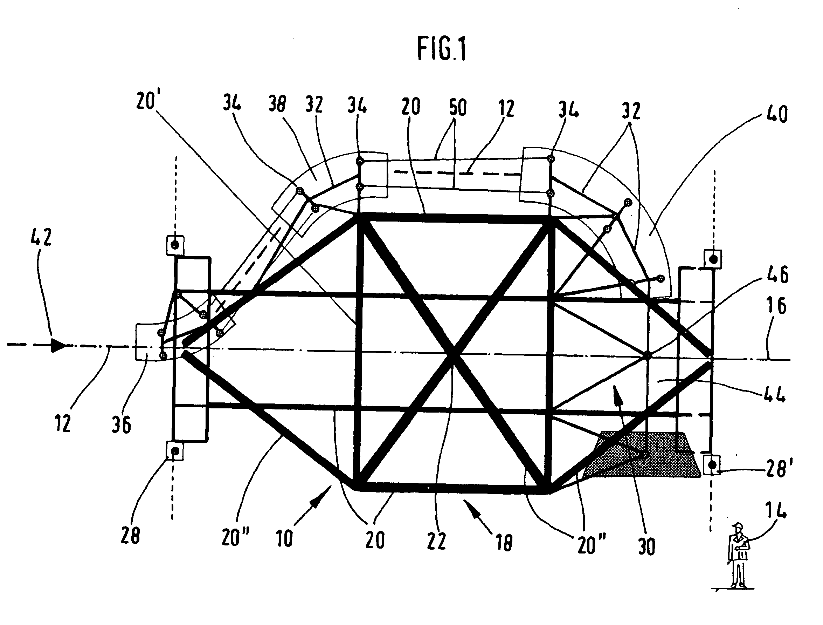

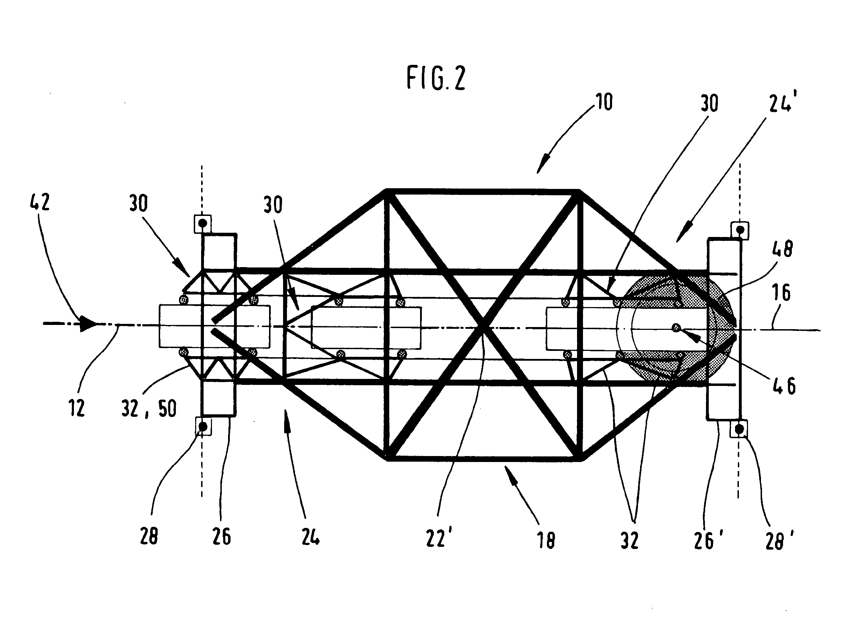

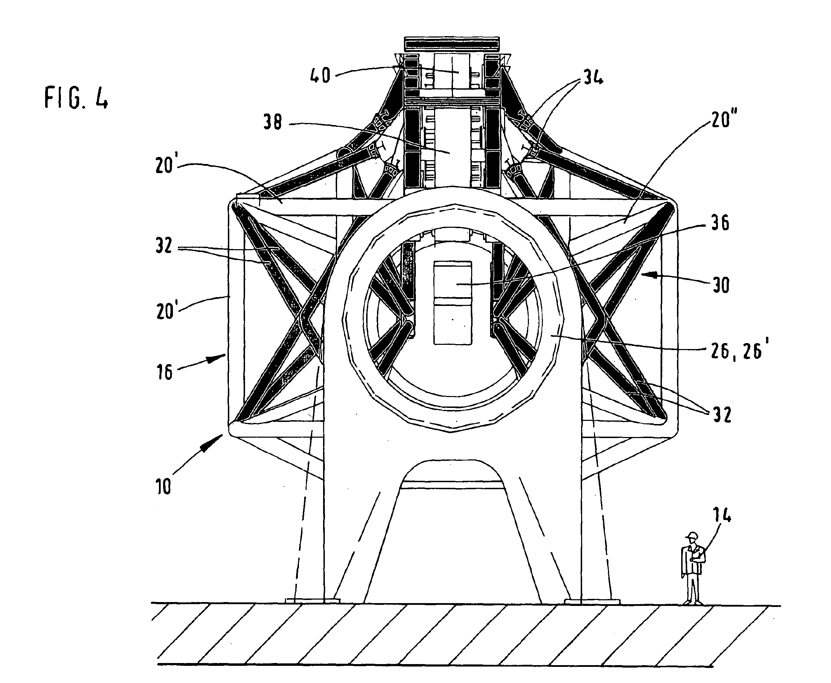

In den Fig. 1, 2 bzw. 3, 4 ist der prinzipielle Aufbau einer Ausführungsform einer solchen erfindungsgemäßen Gantry-Anordnung 10 zur isozentrischen Führung eines Teilchenstrahls, d.h. Ionenstrahls, 12 gezeigt. Dabei sind die Abmessungen und Größenverhältnisse einer solchen Gantry-Anordnung 10 im Vergleich zur Größe eines Menschen 14 veranschaulicht.

Die Gantry-Anordnung 10 ist um eine horizontale Längsachse 16 drehbar und umfasst eine weitestgehend rotationssymmetrische Primärstruktur 18. Die Primärstruktur 18 ist als ein Raumfachwerk aus im Wesentlichen horizontalen Trägern 20, vertikalen Trägern 20' und diagonalen Trägern 20" gebildet. Die diagonalen Träger 20" versteifen zum Beispiel den rechteckigen Rahmen der Primärstruktur 18 und schneiden sind in flächenzentrischen Punkten 22, 22'.

Weiterhin umfasst die Primärstruktur 18 an deren Enden 24, 24' zwei Auflagertragringe 26, 26' in Form von Kastenträgern oder dergleichen, die mit stationären Lagerständern 28, 28' zusammenwirken. Über die kastenförmigen Auflagertragringe 26, 26' wird die Drehverbindung zwischen der Primärstruktur 18 und den stationären Lagerständern 28, 28' hergestellt. Der eine Lagerstand 28 der zwei stationären Lagerständer 28, 28' ist dabei als Loslager ausgebildet. Der andere Lagerstand 28' der zwei stationären Lagerständer 28, 28' ist als Festlager ausgestaltet.

Die von der Primärstruktur 18 getragene Sekundärstruktur 30 ist hinsichtlich Gewicht und Steifigkeit so optimiert, dass die vertikalen Durchbiegungen bzw. Verschiebungen bzw. Verformungen bzw. Deformationen infolge der Magneten 36, 38 und 40 in allen Drehwinkellagen bzw. Drehwinkelpositionen der Gantry-Anordnung 10 näherungsweise gleich groß sind. In bevorzugter Weise ist die Sekundärstruktur 30 steifigkeitsmäßig so ausgebildet, dass diese vertikalen Verschiebungen in sämtlichen Drehwinkellagen der Gantry-Anordnung 10 gleich groß sind.

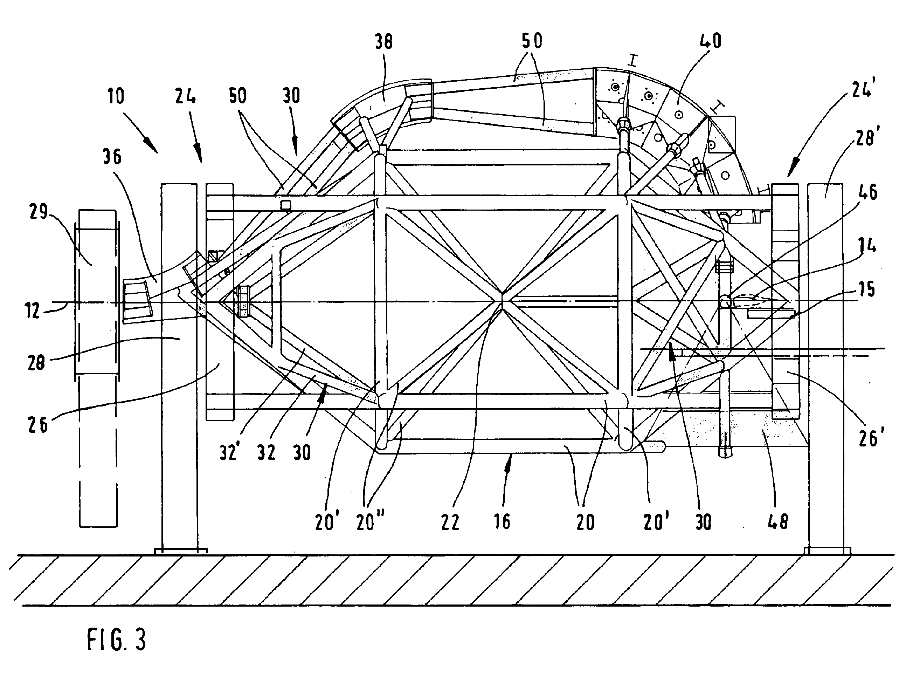

Von der Primärstruktur 18 wird, wie in der Fig. 3 lediglich schematisch angedeutet, eine Kabeldreh- oder Kabelschleppeinrichtung 29 aufgenommen.

Die Primärstruktur 18 stützt die Sekundärstruktur 30 ab. Die Sekundärstruktur 30 wird aus Trägern 32 und Kopplungselementen 34 zur Aufnahme bzw. Halterung und Befestigung von Magneten 36, 38, 40 sowie zwischen den Magneten 36, 38, 40 angeordneten Teilchenstrahlführungselementen (nicht gezeigt) und Stelzen 50 gebildet.

Bei den in den Fig. 1 bis 4 gezeigten Ausführungsbeispielen der Gantry-Anordnung 10 sind jeweils drei Magnete 36, 38, 40 zum Ablenken des Teilchenstrahls 12 vorhanden. Die Magnete 36, 38, 40 lenken den Teilchenstrahl 12 um, der von einem Teilchenbeschleuniger, im vorliegenden Fall also von einem Protonen- bzw. Ionenbeschleuniger (nicht gezeigt), gemäß Pfeil 42 axial eingeschossen wird, so dass er radial, also senkrecht zur horizontalen Längsachse 16 zu einem Patientenraum 44 gelenkt und in den vorgesehenen (Behandlungs-)Zielpunkt 46 triff. Exakt am (Behandlungs-)Zielpunkt 46 kann der Patient 14 somit in horizontaler Lage behandelt werden. Mit dem Patiententisch 15 lässt sich der Patient und damit der Tumor nach drei Raumrichtungen (X-Y-Z) exakt zum Zielpunkt 46 positionieren und, mittels Drehung um die Hochachse Z, in die erforderliche Winkellage bringen. Der in den Patientenraum 44 einmündende Teilchenstrahl 12 wird durch einen Abschirmblock 48 aufgefangen, welcher zugleich ein Gegengewicht für die gesamte Gantry-Anordnung 10 bilden kann.

Im Einzelnen ist ein erster Magnet 36 einem Teilchenbeschleuniger auf der horizontalen Längsachse 16 zugewandt angeordnet. Der erste Magnet 36 dient der Aufnahme des axial eingeschossenen Teilchenstrahls 12 und seiner Ablenkung aus der horizontalen Längsachse 16. Bezogen auf die Radialebene des Strahles dreht der erste Magnet 36 den axial eingeschossenen Teilchenstrahl 12 um einen vorgegebenen Winkel von etwa 30° bis 60°, insbesondere um ungefähr 45° aus der Längsachse 16. Dazu wird der erste Magnet 36 in der horizontalen Längsachse 16 über Stelzen 50' an der Primärstruktur 18 freilagernd bzw. fliegend gehalten, insbesondere ist er quasi freitragend und achsensymmetrisch aufgehängt.

Der zweite Magnet 38 ist zur Aufnahme des von dem ersten Magneten 36 kommenden Teilchenstrahls 12 und zur Rückdrehung des Strahles auf eine Bahn parallel zur horizontalen Längsachse 16 vorgesehen. Er dreht also den Teilchenstrahl um einen vorgegebenen Winkel von etwa 30° bis 60°, insbesondere um ungefähr 45°, wieder zurück bis er erneut parallel zur horizontalen Längsachse verläuft.

Schließlich wird der dritte Magnet 40 zur Aufnahme des von dem zweiten Magneten 38 kommenden Teilchenstrahls 12, zur radialen und senkrecht zu der horizontalen Längsachse 16 erfolgenden Strahlablenkung in den Patientenraum 44 und dem genauen (Behandlungs-)Zielpunkt 46 verwendet. Der dritte Magnet 40 lenkt den Teilchenstrahl 12 radial und im Wesentlichen senkrecht zur horizontalen Längsachse 16 um einen Winkel zwischen etwa 60° bis 120°, insbesondere um ungefähr 90°, ab.

Grundsätzlich können für die Ablenkungen des Teilchenstrahls weniger oder mehr Magnete als hier dargestellt verwendet werden. Insbesondere lassen sich die Magnete 38 und 40 zu einer Baueinheit vereinigen, die den Teilchenstrahl um etwa 120° bis 150° ablenkt.

Die von der Primärstruktur 18 abgestützte, die Magnete 36, 38, 40 halternde Sekundärstruktur 30 ist gegen azimutale Kippbewegungen der Magnete 36, 38, 40 torsionssteif an der Primärstruktur 18 angeordnet.

Die Magnete 36, 38 40 selbst bewegen sich auf Kreisbahnen um eine (imaginäre) Drehachse 52, welche gegenüber der horizontalen Längsachse 16 der Gantry-Anordnung 10 im unbelasteten Zustand verschoben ist. Wie der Größenvergleich zum Menschen 14 zeigt, werden der zweite und dritte Magnet 38, 40 auf beachtlichen Radien geführt. Die Drehachse 52 ist dabei gegenüber der horizontalen Längsachse 16 der Gantry-Anordnung 10 vorzugsweise unter Verwendung der vertikalen elastischen Verschiebungen der Magnete und mittels der Methode der kleinsten Fehlerquadrate bestimmbar.

Die Sekundärstruktur 30, die von der Primärstruktur 18 abgestützt wird und die Magnete 36, 38, 40 aufnimmt und haltert, ist darüber hinaus gegenüber der Primärstruktur 18 vergleichsweise nachgiebig bzw. flexibel ausgebildet.

Auf Grund der hohen Gewichte der Magnete 36, 38, 40 einschließlich der Teilchenstrahlführungselemente sowie der immer vorhandenen Elastizitäten von Tragstrukturen ganz allgemein kommt es zu einem Durchhang der Gantry-Anordnung 10, der zudem je nach Drehwinkelposition unterschiedlich groß ausfällt. Hinzu kommt, dass die Verformungen in verschiedenen Drehwinkelpositionen der Gantry-Anordnung 10 unterschiedlich sind.

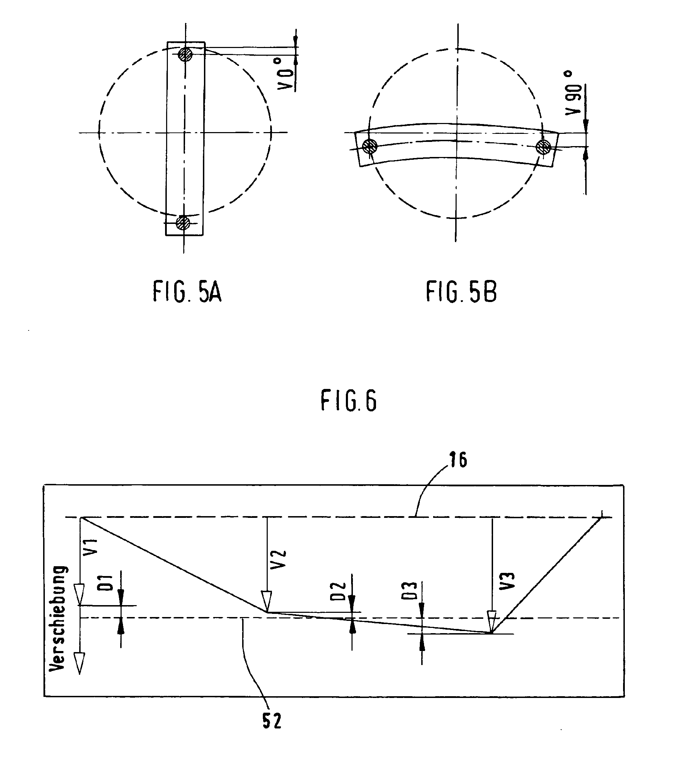

Die Fig. 5A und 5B veranschaulichen diese Verformungen in zwei unterschiedlichen Gantry-Stellungen für die Magnete und das Gegengewicht 48. Um in beliebigen Drehwinkelpositionen der Gantry-Anordnung 10 möglichst gleiche vertikale Verschiebungen V2, V3 der Magnete 38, 40 realisieren zu können, ist es erforderlich, sich an ihren maximalen Verschiebungen zu orientieren. Diese ergeben sich in den horizontalen Lagen -90° und +90° der Magnete 38, 40 infolge großer Hebelwirkungen auf die Gantry-Anordnung 10. Mit Blick auf diese maximalen Verschiebungen wird die Sekundärstruktur 30 in vertikaler Richtung entsprechend weich und azimutal dazu steif ausgelegt, um unerwünschte Kippbewegungen der Magnete 36, 38, 40 auszuschließen.

Die Gantry-Anordnung 10 nach der Erfindung stellt sicher, dass der Teilchenstrahl 12 ohne korrigierenden Zugriff auf die Felder der Magneten bzw. der Teilchenstrahlführungselemente stets in ein und denselben (Behandlungs-)Zielpunkt 46 (auf)trifft, unabhängig davon, in welcher Drehwinkellage die Magnete 36, 38, 40 stehen.

Damit der Teilchenstrahl 12 in allen Drehwinkellagen der Magnete 36, 38, 40 mit möglichst hoher Präzision in den (Behandlungs-)Zielpunkt 46 trifft, führt die Gantry-Anordnung 10, trotz elastischer Deformationsverschiebungen, die Magnete 36, 38, 40 um die gemeinsame horizontale Drehachse 52 auf Kreisbahnen, dergestalt, dass sich deren relative Positionen zueinander nicht verschieben. Der ursprünglich horizontale Teilchenstrahl wird um exakt 90° abgelenkt. Der Teilchenstrahl 12 liegt mithin in der Kreisebene des dritten Magneten 40 und trifft in allen Drehwinkellagen stets in deren Kreismittelpunkt, den (Behandlungs-)Zielpunkt 46.

Die konstruktive Ausgestaltung der Gantry-Anordnung 10 nach der Erfindung basiert dabei auf folgender Erkenntnis:
Zunächst wurde versucht, eine Gantry-Anordnung, entsprechend aus dem Stand der Technik allgemein bekannten Grundsätzen, so steif wie möglich auszulegen. Anhand von Simulationsrechnungen wurde jedoch festgestellt, dass sich die vertikalen Verschiebungen V1, V2, V3 der drei Magnete 36, 38, 40 wegen des dabei anwachsenden Eigengewichtes der Gantry-Anordnung 10 insgesamt nicht beliebig reduzieren lassen.

Um die erforderliche Zielgenauigkeit zu erreichen und gleichzeitig das Eigengewicht und somit die Kosten zu reduzieren, wurde die isokinetische Gantry entwickelt. Sie beinhaltet eine ideale Struktur, die unter der Last der Magneten 36, 38, 40, einschließlich ihres Eigengewichtes, in allen Drehwinkelpositionen gleiche Deformationsverschiebungen (V1 = V2 = V3 = VH) aufweist. Damit bewegen sich die Magnete 36, 38, 40 auf Kreisbahnen um die Achse 52, die um den Betrag VH gegenüber der Längsachse 16 der unbelasteten Lager 28, 28' verschoben ist.

Außerdem trifft bei der erfindungsgemäßen Gantry-Anordnung 10 der einmal ausgerichtete Teilchenstrahl 12 in allen Drehwinkellagen in ein und denselben (Behandlungs-)Zielpunkt 46, vorausgesetzt, dass die Magnete 36, 38, 40 in azimutaler Richtung gegen Kippbewegungen quasi starr mit der Gantry-Struktur 10 verbunden sind. Dieser (Behandlungs-)Zielpunkt 46 ist der Schnittpunkt zwischen der Drehachse 52 und dem um 90° umgelenkten radialen Teilchenstrahl 12. Bezogen auf diesen Schnittpunkt, weist die Gantry-Anordnung 10 keine Deformationsverschiebungen auf.

Da in Bezug auf die horizontale Achse 52 nur die Relativverschiebungen der Teilchenstrahlführungselemente zueinander maßgeblich sind und nicht die Absolutverschiebungen, können ohne weiteres betragsmäßig größere Absolutverschiebungen zugelassen werden. Hierdurch ergeben sich beachtliche Werkstoffeinsparungen, da gewisse Strukturbereiche relativ dünnwandig ausgeführt werden können. Gegenüber einer steifigkeitsausgelegten Gantry hat die erfindungsgemäße Gantry-Anordnung 10 einen bedeutend geringeren Stahlbedarf.

In der Fig. 6 ist ein Beispiel für die realisierten Verschiebungen V1, V2, V3 der drei Magnete 36, 38, 40 samt der Drehachse 52 dargestellt. Da die Verschiebung V1 des weich aufgehängten ersten Magneten 36 in allen Drehwinkelpositionen der Gantry-Anordnung 10 bei symmetrischer Einspannung praktisch gleich groß und konstruktiv leicht bestimmbar ist, wird bei waagerechter Drehachse 52 die Verschiebung V1 zur weiteren Optimierung so gewählt, dass sie annähernd (V2 + V3)/2 gleicht.

Damit sich die Magnete 36, 38, 40 in Achsrichtung gesehen auf Kreis- und nicht etwa auf Ellipsenbahnen bewegen, sollen die Verschiebungen V2 und V3 ferner möglichst gleich groß (aus-) gewählt sein, was sich im Rahmen einer Feinoptimierung für mehrere Drehwinkelpositionen realisieren lässt.

Entsprechend der Fig. 6 wird die Lage der horizontalen Drehachse 52 zum Beispiel nach dem Prinzip der kleinsten Fehlerquadrate bezüglich der Abstände D1, D2 und D3 gewählt. Auf diese Weise liegt sie zwischen V1, V2 und V3.

Die Erfindung ist nicht auf die dargestellten Ausführungsformen beschränkt. So ist es ohne weiteres möglich, die erfindungsgemäße isokinetische Gantry-Anordnung 10 ebenso zur Behandlung mittels Protonenstrahlen einzusetzen. Dabei ist die konstruktive Ausbildung einer solchen Gantry-Anordnung im Wesentlichen identisch. Lediglich die Größenverhältnisse verändern sich infolge unterschiedlichen Gewichtes und Abmessung der Magnete und Teilchenstrahlführungselemente. Ohne im Einzelnen dargestellt zu sein, ist es ebenso denkbar, mit der erfindungsgemäßen Gantry-Anordnung 10 nicht nur drei Magnete 36, 38, 40, sondern weniger oder mehr als drei Magnete abzustützen. So ist es beispielsweise möglich den zweiten und dritten Magneten baulich als Einheit zusammenzufassen.

### Bezugszeichenliste

- 10: Gantry-Anordnung
- 12: Teilchenstrahl
- 14: Mensch, Patient
- 15: Patiententisch
- 16: horizontale Längsachse
- 18: Primärstruktur
- 20: horizontaler Träger
- 20': vertikaler Träger
- 20": diagonaler Träger
- 22, 22': flächenzentrischer Punkt
- 24, 24': Ende der Primärstruktur
- 26, 26': Auflagertragringe
- 28, 28': stationäre Lagerständer
- 29: Kabeldreh-/Kabelschleppeinrichtung
- 30: Sekundärstruktur
- 32, 32': Träger
- 34: Kopplungselement
- 36: erster Magnet
- 38: zweiter Magnet
- 40: dritter Magnet
- 42: Pfeil
- 44: Patientenraum
- 46: (Behandlungs-)Zielpunkt
- 48: Abschirmblock
- 50, 50': Stelzen
- 52: Drehachse
- D1, D2, D3: Abstände
- V1, V2, V3: Verschiebungen.

## Patentansprüche

1. Gantry-Anordnung zur isozentrischen Führung eines Teilchenstrahls (12), die um eine horizontale Längsachse (16) drehbar ist und eine Strahlenoptik, mit Magneten (36, 38, 40), welche den von einem Teilchenbeschleuniger axial eingeschossenen Teilchenstrahl (12) radial und senkrecht zur horizontalen Längsachse (16) ablenkt, sowie eine weitgehend rotationssymmetrische Primärstruktur (18) aufweist, **gekennzeichnet durch** eine von der Primärstruktur (18) abgestützte, die Magnete (36, 38, 40) halternde Sekundärstruktur (30), wobei die Magnete (36, 38, 40) auf Kreisbahnen um eine Drehachse (52), welche gegenüber der horizontalen Längsachse (16) der Gantry-Anordnung (10) im unbelasteten Zustand verschoben ist, bewegbar sind und wobei die Sekundärstruktur (30) eine Steifigkeit aufweist, die derart bemessen ist, dass vertikale Verschiebungen der Magnete (36, 38, 40) infolge der Belastung der Gantry-Anordnung (10) mit ihrem Gewicht in sämtlichen Drehwinkellagen im Wesentlichen gleich groß bzw. isokinetisch sind.

2. Gantry-Anordnung nach Anspruch 1, **dadurch gekennzeichnet, dass** die Sekundärstruktur (30) eine Steifigkeit aufweist, die derart bemessen ist, dass vertikale Verschiebungen der Magnete (36, 38, 40) infolge der Belastung der Gantry-Anordnung (10) mit ihrem Gewicht in sämtlichen Drehwinkellagen gleich groß sind.

3. Gantry-Anordnung nach Anspruch 1 oder 2, **dadurch gekennzeichnet, dass** als Bestrahlungs-Zielpunkt der Schnittpunkt zwischen dem Ionen-/Protonen-Strahl und der lastverschobenen Drehachse (52) dient, die gegenüber der horizontalen Längsachse (16) der Gantry-Anordnung (10) im unbelasteten Zustand mittels der Methode der kleinsten Fehlerquadrate aus Lastverschiebungen der Magnete (36, 38, 40) bestimmt worden ist.

4. Gantry-Anordnung nach einem der Ansprüche 1 bis 3, **dadurch gekennzeichnet, dass** die Primärstruktur (18) als Raumfachwerk aus im Wesentlichen horizontalen und vertikalen Trägern (20, 20') sowie diagonalen Trägern (20") ausgebildet ist.

5. Gantry-Anordnung nach Anspruch 4, **dadurch gekennzeichnet, dass** einige der diagonalen Träger (20'') rechteckige Rahmen der Primärstruktur (18) aussteifen und sich in flächenzentrischen Punkten (22, 22') schneiden.

6. Gantry-Anordnung nach einem der Ansprüche 1 bis 5, **dadurch gekennzeichnet, dass** die Primärstruktur (18) an deren Enden (24, 24') zwei, insbesondere kastenförmige, Auflagertragringe (26, 26') umfasst, die mit stationären Lagerständern (28, 28') zusammenwirken.

7. Gantry-Anordnung nach Anspruch 6, **dadurch gekennzeichnet, dass** einer (28) der zwei stationären Lagerständer (28, 28') als Loslager und der andere (28') der zwei stationären Lagerständer (28, 28') als Festlager ausgebildet ist.

8. Gantry-Anordnung nach einem der Ansprüche 1 bis 7, **dadurch gekennzeichnet, dass** die von der Primärstruktur (18) abgestützte, die Magnete (36, 38, 40) halternde Sekundärstruktur (30) gegenüber der Primärstruktur (18) weniger steif ausgebildet ist.

9. Gantry-Anordnung nach einem der Ansprüche 1 bis 8, **dadurch gekennzeichnet, dass** die von der Primärstruktur (18) abgestützte, die Magnete (36, 38, 40) halternde Sekundärstruktur (30) gegen azimutale Kippbewegungen der Magnete (36, 38, 40) torsionssteif an der Primärstruktur (18) angeordnet ist.

10. Gantry-Anordnung nach einem der Ansprüche 1 bis 9, **dadurch gekennzeichnet, dass** die von der Primärstruktur (18) abgestützte Sekundärstruktur (30) wenigstens einen, insbesondere drei, Magnete (36, 38, 40) aufnimmt.

11. Gantry-Anordnung nach einem der Ansprüche 1 bis 10, **dadurch gekennzeichnet, dass** ein erster Magnet (36) zur Aufnahme des axial eingeschossenen Teilchenstrahls (12) und zur Ablenkung aus der horizontalen Längsachse (16) im Wesentlichen auf der horizontalen Längsachse (16) dem Teilchenbeschleuniger zugewandt angeordnet ist.

12. Gantry-Anordnung nach Anspruch 11, **dadurch gekennzeichnet, dass** der erste Magnet (36) den axial eingeschossenen Teilchenstrahl (12) aus der horizontalen Längsachse (16) um einen Winkel etwa zwischen 30° bis 60°, insbesondere von ungefähr 45°, ablenkt.

13. Gantry-Anordnung nach Anspruch 11 oder 12, **dadurch gekennzeichnet, dass** der erste Magnet (36) auf der horizontalen Längsachse (16) über lange Träger (32') an der Primärstruktur (18) freitragend angeordnet ist.

14. Gantry-Anordnung nach einem der Ansprüche 1 bis 13, **dadurch gekennzeichnet, dass** ein zweiter Magnet (38) zur Aufnahme des von dem ersten Magnet (36) kommenden Teilchenstrahls (12) vorgesehen ist, der ihn parallel zur horizontalen Längsachse (16) ablenkt und der über Stelzen (50) an den ersten Magneten angekoppelt ist.

15. Gantry-Anordnung nach Anspruch 14, **dadurch gekennzeichnet, dass** der zweite Magnet (38) den von dem ersten Magnet (36) abgelenkten Teilchenstrahl (12) parallel zu der horizontalen Längsachse (16) um einen Winkel etwa zwischen 30° bis 60°, insbesondere von ungefähr 45°, zurücklenkt.

16. Gantry-Anordnung nach einem der Ansprüche 14 bis 15, **dadurch gekennzeichnet, dass** ein dritter Magnet (40) zur Aufnahme des von dem zweiten Magnet (38) kommenden Teilchenstrahls (12) vorgesehen ist, der ihn radial und senkrecht zu der horizontalen Längsachse (16) ablenkt und der über Stelzen (50) an den zweiten Magneten angekoppelt ist.

17. Gantry-Anordnung nach Anspruch 16, **dadurch gekennzeichnet, dass** der dritte Magnet (40) den von dem zweiten Magnet (38) kommenden Teilchenstrahl (12) radial und im Wesentlichen senkrecht zu der horizontalen Längsachse (16) um einen Winkel etwa zwischen 60° bis 120°, insbesondere von ungefähr 90°, ablenkt.

18. Gantry-Anordnung nach einem der Ansprüche 1 bis 17, **dadurch gekennzeichnet, dass** die Magnete (36, 38, 40) zur Aufnahme und zur Ablenkung eines Teilchenstrahls (12) aus Protonen ausgebildet sind.

19. Gantry-Anordnung nach einem der Ansprüche 1 bis 17, **dadurch gekennzeichnet, dass** die Magnete (36, 38, 40) zur Aufnahme und zur Ablenkung eines Teilchenstrahls (12) aus Ionen ausgebildet sind.

20. Gantry-Anordnung nach einem der Ansprüche 1 bis 19, **dadurch gekennzeichnet, dass** zwischen den Magneten (36, 38, 40) Teilchenstrahlführungselemente angeordnet sind.

21. Gantry-Anordnung nach einem der Ansprüche 16 bis 20, **dadurch gekennzeichnet, dass** der zweite und dritte Magnet eine integrierte Baueinheit bilden.

22. Verfahren zur Auslegung einer Gantry-Anordnung (10) für die isozentrische Führung eines Teilchenstrahls (12) nach einem der vorhergehenden Ansprüche, mittels welchem die Steifigkeit einer im Wesentlichen rotationssymmetrischen Primärstruktur (18) derart bemessen wird, dass die vertikalen Verschiebungen der Magnete (36, 38, 40) infolge ihres Gewichtes in sämtlichen Drehwinkellagen der Gantry-Anordnung (10) im Wesentlichen gleich groß, vorzugsweise gleich groß sind, wobei die Magnete (36, 38, 40) auf Kreisbahnen um eine Drehachse (52), welche gegenüber der horizontalen Längsachse (16) der Gantry-Anordnung (10) im unbelasteten Zustand verschoben ist, bewegt werden.

23. Verfahren nach Anspruch 22, **dadurch gekennzeichnet, dass** die durch Lasten abgesenkte theoretische Drehachse (52) gegenüber der horizontalen Längsachse (16) der Gantry-Anordnung (10) im unbelasteten Zustand mittels der Methode der kleinsten Fehlerquadrate bestimmt wird und als Bestrahlungs-Zielpukt der "Schnittpunkt" zwischen dem Teilchenstrahl und der lastverschobenen theoretischen Drehachse (52) definiert ist.

## Claims

1. Gantry arrangement for the isocentric guidance of a particle beam (12) that can be rotated about a horizontal longitudinal axis (16) and has a beam optical system with magnets (36, 38, 40) that radially and vertically relative to the horizontal longitudinal axis (16) deflect a particle beam (12) that has been axially injected by a particle beam accelerator, and having an extensive rotationally-symmetrical primary structure (18), **characterized by** a secondary structure (30) supported by the primary structure (18) and holding the magnets (36, 38, 40), with the magnets (36, 38, 40) can move on circular tracks about an axis of rotation (52) that is displaced in the unloaded state relative to the horizontal longitudinal axis (16) of the gantry arrangement (10) and with the secondary structure (30) having a rigidity that is designed such that vertical displacements of the magnets (36, 38, 40) due to the load of the gantry arrangement (10) with the weight thereof are essentially of the same magnitude and isokinetic, respectively, in all angle of rotation positions.

2. Gantry arrangement in accordance with claim 1, **characterized in that**, the secondary structure (30) has a rigidity that is designed such that vertical displacements of the magnets (36, 38, 40) due to the load of the gantry arrangement (10) with the weight thereof are of equal magnitude in all angle of rotation positions.

3. Gantry arrangement in accordance with claim 1 or 2, **characterized in that**, as a radiation point of aim, the intersection point between the ion/proton beam and the load-displaced axis of rotation (52) is used, that with respect to the horizontal longitudinal axis (16) of the gantry arrangement (10) in the unloaded state has been determined from the load displacements of the magnets (36, 38, 40) using the method of the smallest error squares.

4. Gantry arrangement in accordance with one of claims 1 to 3, **characterized in that**, the primary structure (18) is designed as a three-dimensional framework consisting essentially of horizontal and vertical members (20, 20') and diagonal members (20").

5. Gantry arrangement in accordance with claim 4, **characterized in that**, some of the diagonal members (20") stiffen rectangulars frames of the primary structure (18) and intersect in the centre points (22, 22') of the surface.

6. Gantry arrangement in accordance with one of claims 1 to 5, **characterized in that**, the primary structure (18) has at its ends (24, 24') two, particularly box-shaped, member support rings (26, 26') that interact with fixed bearing pedestals (28, 28').

7. Gantry arrangement in accordance with claim 6, **characterized in that**, one (28) of the two fixed bearing pedestals (28, 28') is designed as a floating bearing and the other (28') of the two fixed bearing pedestals (28, 28') is designed as a fixed bearing.

8. Gantry arrangement in accordance with one of claims 1 to 7, **characterized in that**, the secondary structure (30) holding the magnets (36, 38, 40) and supported by the primary structure (18) is of a less-rigid design than the primary structure (18).

9. Gantry arrangement in accordance with one of claims 1 to 8, **characterized in that**, the secondary structure (30) holding the magnets (36, 38, 40) and supported by the primary structure (18) is attached to the primary structure (18) so as to be torsionally stiff with respect to tilting movements of the magnets (36, 38, 40) in azimuth.

10. Gantry arrangement in accordance with one of claims 1 to 9, **characterized in that**, the secondary structure (30) supported by the primary structure (18) has at least one, in particular three, magnet(s) (36, 38, 40).

11. Gantry arrangement in accordance with one of claims 1 to 10, **characterized in that**, a first magnet (36) is essentially arranged on the horizontal longitudinal axis (16) facing towards the particle accelerator, to take the axially injected particle beam (12) and to deflect it from the horizontal longitudinal axis (16).

12. Gantry arrangement in accordance with claim 11, **characterized in that**, the first magnet (36) deflects the axially injected particle beam (12) from the horizontal longitudinal axis (16) by an angle of approximately between 30° to 60°, in particular of approximately 45°.

13. Gantry arrangement in accordance with claim 11 or 12, **characterized in that**, the first magnet (36) is cantilever mounted on the primary structure (18) on the horizontal longitudinal axis (16) above long members (32').

14. Gantry arrangement in accordance with one of claims 1 to 13, **characterized in that**, a second magnet (38) is provided to take the particle beam (12), coming from the first magnet (36), and deflect it parallel to the horizontal longitudinal axis (16), and is coupled to the first magnet by struts (50).

15. Gantry arrangement in accordance with claim 14, **characterized in that**, the second magnet (38) deflects the particle beam (12), deflected by the first magnet (36), back parallel to the horizontal longitudinal axis (16) by an angle of approximately between 30° to 60°, in particular by approximately 45°.

16. Gantry arrangement in accordance with one of claims 14 to 15, **characterized in that**, a third magnet (40) is provided to take the particle beam (12), coming from the second magnet (38), that deflects it radial and vertical to the horizontal longitudinal axis (16) and is coupled to the second magnet by struts (50).

17. Gantry arrangement in accordance with claim 16, **characterized in that**, the third magnet (40) deflects the particle beam (12), coming from the second magnet (38), radial and essentially vertical to the horizontal longitudinal axis (16) by an angle of approximately between 60° to 120°, in particular by approximately 90°.

18. Gantry arrangement in accordance with one of claims 1 to 17, **characterized in that**, the magnets (36, 38, 40) are designed to take and deflect a particle beam (12) of protons.

19. Gantry arrangement in accordance with one of claims 1 to 17, **characterized in that**, the magnets (36, 38, 40) are designed to take and deflect a particle beam (12) of ions.

20. Gantry arrangement in accordance with one of claims 1 to 19, **characterized in that**, particle beam guidance elements are arranged between the magnets (36, 38, 40).

21. Gantry arrangement in accordance with one of claims 16 to 20, **characterized in that**, the second and third magnets form an integrated unit.

22. Method for design of a gantry arrangement (10) for the isocentric guidance of a particle beam (12) in accordance with one of the preceding claims, by means of which the rigidity of a primary structure (18) that is essentially rotationally-symmetrical is designed in such a way that the vertical displacements of the magnets (36, 38, 40) due to their weight are essentially of equal magnitude, preferably of exactly equal magnitude, in all the angle of rotation positions of the gantry arrangement (10), with the magnets (36, 38, 40) moving on circular tracks about an axis of rotation (52) that is displaced with respect to the horizontal longitudinal axis (16) of the gantry arrangement (10) in the unloaded state.

23. Method in accordance with claim 22, **characterized in that**, the theoretical axis of rotation (52) lowered due to loads is determined with respect to the horizontal longitudinal axis (16) of the gantry arrangement (10) in the unloaded state by means of the method of smallest error squares and the "intersection point" between the particle beam and the load-displaced theoretical axis of rotation (52) is defined as the radiation point of aim.

## Revendications

1. Système de portique pour le guidage isocentrique d'un faisceau de particules (12), qui est orientable autour d'un axe longitudinal horizontal (16) et présente une optique géométrique avec des aimants (36, 38, 40) qui dévie, de manière radiale et perpendiculaire par rapport à l'axe longitudinal horizontal (16), le faisceau de particules (12) injecté de manière axiale par un accélérateur de particules, ainsi qu'une structure primaire sensiblement à symétrie de révolution (18), **caractérisé par** une structure secondaire (30) tenant les aimants (36, 38, 40) et supportée par la structure primaire (18), les aimants (36, 38, 40) étant déplaçables sur des trajectoires circulaires autour d'un axe de rotation (52) qui est décalé par rapport à l'axe longitudinal horizontal (16) du système de portique (10) à l'état non chargé, et la structure secondaire (30) présentant une rigidité qui est calculée de sorte que les décalages verticaux des aimants (36, 38, 40) résultant de la charge du système de portique (10) par leur poids soient essentiellement identiques ou isocinétiques dans toutes les positions d'angle de rotation.

2. Système de portique selon la revendication 1, **caractérisé en ce que** la structure secondaire (30) présente une rigidité qui est calculée de sorte que les décalages verticaux des aimants (36, 38, 40) résultant de la charge du système de portique (10) par leur poids sont identiques dans toutes les positions d'angle de rotation.

3. Système de portique selon la revendication 1 ou 2, **caractérisé en ce qu'**est défini comme point de destination du rayonnement le point d'intersection entre le faisceau d'ions/de protons et l'axe de rotation décalé par les charges (52), qui a été déterminé par rapport à l'axe longitudinal horizontal (16) du système de portique (10) à l'état non chargé au moyen de la méthode du plus petit carré des erreurs à partir des décalages des aimants (36, 38, 40) dus aux charges.

4. Système de portique selon l'une des revendications 1 à 3, **caractérisé en ce que** la structure primaire (18) est formée comme treillis à trois dimensions de poutrelles essentiellement horizontales et verticales (20, 20') ainsi que de poutrelles diagonales (20").

5. Système de portique selon la revendication 4, **caractérisé en ce que** certaines des poutrelles diagonales (20") renforcent des cadres rectangulaires de la structure primaire (18) et se coupent en des points centraux des faces (22, 22').

6. Système de portique selon l'une des revendications 1 à 5, **caractérisé en ce que** la structure primaire (18) comprend à ses extrémités (24, 24') deux anneaux de retenue d'appui, en particulier en forme de boîte (26, 26'), qui coopèrent avec des supports palier stationnaires (28, 28').

7. Système de portique selon la revendication 6, **caractérisé en ce que** l'un (28) des deux supports palier stationnaires (28, 28') est formé comme palier libre et l'autre (28') des deux supports palier stationnaires (28, 28') est formé comme palier fixe.

8. Système de portique selon l'une des revendications 1 à 7,
**caractérisé en ce que** la structure secondaire (30) tenant les aimants (36, 38, 40) et supportée par la structure primaire (18) est formée de manière moins rigide par rapport à la structure primaire (18).

9. Système de portique selon l'une des revendications 1 à 8, **caractérisé en ce que** la structure secondaire (30) tenant les aimants (36, 38, 40) et supportée par la structure primaire (18) est disposée par rapport à la structure primaire (18) de manière résistante à la torsion afin d'éviter tout mouvement de basculement azimutal des aimants (36, 38, 40).

10. Système de portique selon l'une des revendications 1 à 9, **caractérisé en ce que** la structure secondaire (30) supportée par la structure primaire (18) reçoit au moins un, en particulier trois aimants (36, 38, 40).

11. Système de portique selon l'une des revendications 1 à 10, **caractérisé en ce qu'**un premier aimant (36) pour la réception du faisceau de particules (12) injecté de manière axiale et pour la déviation par rapport à l'axe longitudinal horizontal (16) est disposé de façon à être orienté vers l'accélérateur de particules essentiellement sur l'axe longitudinal horizontal (16).

12. Système de portique selon la revendication 11, **caractérisé en ce que** le premier aimant (36) dévie le faisceau de particules (12) injecté de manière axiale par rapport à l'axe longitudinal horizontal (16) d'un angle compris environ entre 30° et 60° , en particulier de 45° environ.

13. Système de portique selon la revendication 11 ou 12, **caractérisé en ce que** le premier aimant (36) sur l'axe longitudinal horizontal (16) est disposé en porte-à-faux au niveau de la structure primaire (18) via de longues poutrelles (32').

14. Système de portique selon l'une des revendications 1 à 13, **caractérisé en ce qu'**est prévu un deuxième aimant (38) pour la réception du faisceau de particules (12) provenant du premier aimant (36), qui le dévie de manière parallèle à l'axe longitudinal horizontal (16) et est couplé au premier aimant via des béquilles (50).

15. Système de portique selon la revendication 14, **caractérisé en ce que** le deuxième aimant (38) renvoie le faisceau de particules (12) dévié par le premier aimant (36) en le déviant de manière parallèle à l'axe longitudinal horizontal (16) d'un angle compris environ entre 30° et 60°, en particulier d'environ 45°.

16. Système de portique selon l'une des revendications 14 à 15, **caractérisé en ce qu'**est prévu un troisième aimant (40) pour la réception du faisceau de particules (12) provenant du deuxième aimant (38), qui le dévie de manière radiale et perpendiculaire à l'axe longitudinal horizontal (16) et est couplé au deuxième aimant via des béquilles (50).

17. Système de portique selon la revendication 16, **caractérisé en ce que** le troisième aimant (40) dévie le faisceau de particules (12) provenant du deuxième aimant (38) de manière radiale et essentiellement perpendiculaire à l'axe longitudinal horizontal (16) d'un angle compris environ entre 60° et 120°, en particulier de 90° environ.

18. Système de portique selon l'une des revendications 1 à 17, **caractérisé en ce que** les aimants (36, 38, 40) sont formés pour la réception et la déviation d'un faisceau de particules (12) constitué de protons.

19. Système de portique selon l'une des revendications 1 à 17, **caractérisé en ce que** les aimants (36, 38, 40) sont formés pour la réception et la déviation d'un faisceau de particules (12) constitué d'ions.

20. Système de portique selon l'une des revendications 1 à 19, **caractérisé en ce que** des éléments de guidage du faisceau de particules sont disposés entre les aimants (36, 38, 40).

21. Système de portique selon l'une des revendications 16 à 20, **caractérisé en ce que** le deuxième et le troisième aimants forment une unité de construction intégrée.

22. Procédé de conception d'un système de portique (10) pour le guidage isocentrique d'un faisceau de particules (12) selon l'une des revendications précédentes, au moyen duquel la rigidité d'une structure primaire (18) essentiellement à symétrie de révolution est calculée de telle sorte que les décalages verticaux des aimants (36, 38, 40) résultant de leur poids sont essentiellement identiques et sont de préférence identiques dans toutes les positions d'angle de rotation du système de portique (10), les aimants (36, 38, 40) étant décalés sur des trajectoires circulaires autour d'un axe de rotation (52) qui est décalé par rapport à l'axe longitudinal horizontal (16) du système de portique (10) à l'état non chargé.

23. Procédé selon la revendication 22, **caractérisé en ce que** l'axe de rotation théorique (52) dévié par des charges est déterminé par rapport à l'axe longitudinal horizontal (16) du système de portique (10) à l'état non chargé au moyen de la méthode du plus petit carré des erreurs et **en ce qu'**est défini comme point de destination du rayonnement le « point d'intersection » entre le faisceau de particules et l'axe de rotation théorique décalé par les charges (52).
